# EUROPÄISCHE PATENTANMELDUNG

(11) **EP 3 622 924 A1**
(43) Veröffentlichungstag der Anmeldung: **18.03.2020**
(21) Anmeldenummer: 19195799.2
(22) Anmeldetag: 06.09.2019
(51) Int. Cl.: A61F 9/007, A61F 9/008, A61B 34/00, A61B 17/00

(54) **VERFAHREN ZUR STEUERUNG EINES AUGENCHIRURGISCHEN GERÄTESYSTEMS SOWIE EIN AUGENCHIRURGISCHES GERÄTESYSTEM**

(30) Priorität: 11.09.2018 DE 102018215423; 20.09.2018 DE 102018216085
(71) Anmelder: Geuder AG, 69126 Heidelberg (DE)
(72) Erfinder: FRAUENFELD, Dieter, 69121 Heidelberg (DE); WOLSCHENDORF, Marc, 69198 Schriesheim (DE); STEIN, Marco, 69126 Heidelberg (DE); DRAHEIM, René, 69207 Sandhausen (DE); RUDOLPH, Ulrich, 06112 Halle (DE); GEUDER, Volker, 69126 Heidelberg (DE)
(74) Vertreter: Patent- und Rechtsanwälte Ullrich & Naumann

(57) **Zusammenfassung**

Verfahren zur Steuerung eines augenchirurgischen Gerätesystems, das eine Konsole (1) umfasst, die mindestens eine Recheneinrichtung (2) und mindestens eine graphische Anzeigeeinrichtung (3) aufweist, wobei mindestens ein chirurgisches Handgerät (4) durch das Gerätesystem betreibbar und versorgbar ist, insbesondere mit Überdruck und/oder Unterdruck und/oder Ultraschallleistung und/oder Flüssigkeit und/oder Licht und/oder elektromagnetischer Energie und/oder elektrischer Energie, mit folgenden Verfahrensschritten:
- Anzeigen mindestens eines Benutzerprofils (16) innerhalb eines ersten Darstellungsmoduls (15) auf der Anzeigeeinrichtung (3), wobei dem Benutzerprofil (16) mindestens ein Operationsprofil (17) zugeordnet ist,
- Auswahl des gewünschten Benutzerprofils (16),
- Anzeige mindestens eines dem ausgewählten Benutzerprofil (16) zugeordneten Operationsprofils (17) innerhalb eines zweiten Darstellungsmoduls (18) auf der Anzeigeeinrichtung (3), wobei dem Operationsprofil (17) mindestens ein vordefiniertes Operationselement (19) zugeordnet ist,
- Auswahl eines Operationsprofils (17),
- Anzeige mindestens eines dem Operationsprofil (17) zugeordneten Operationselements (19) innerhalb eines dritten Darstellungsmoduls (20) auf der Anzeigeeinrichtung (3), wobei dem Operationselement (19) mindestens ein Grundparameter des Gerätesystems zugeordnet ist,
- Auswahl eines Operationselementes (19), wobei der mindestens eine Grundparameter eingestellt bzw. berücksichtigt wird.

Des Weiteren ist eine augenchirurgische Vorrichtung zur Durchführung des Verfahrens beansprucht.

## Beschreibung

Die Erfindung betrifft ein Verfahren zur Steuerung eines augenchirurgischen Gerätesystems, das eine Konsole umfasst, die mindestens eine Recheneinrichtung und mindestens eine graphische Anzeigeeinrichtung aufweist, wobei mindestens ein chirurgisches Handgerät durch das Gerätesystem betreibbar und versorgbar ist, insbesondere mit Überdruck und/oder Unterdruck und/oder Ultraschallleistung und/oder Flüssigkeit und/oder Licht und/oder elektromagnetischer Energie und/oder elektrischer Energie. Des Weiteren betrifft die Erfindung ein solches augenchirurgisches Gerätesystem.

Augenchirurgische Gerätesysteme der in Rede stehenden Art sind bereits seit Jahren aus der Praxis bekannt. Diese dienen zur Durchführung von ophthalmologischen Operationen, bspw. einer Phakoemulsifikation. Dabei wird eine durch einen grauen Star getrübte Linse anhand einer mit Ultraschall beaufschlagten Nadel zertrümmert. Die Linsentrümmer werden durch die Nadel hindurch abgesaugt. Um ein Kollabieren sowie eine Überhitzung des Auges zu vermeiden, wird dem Auge während des Eingriffs eine Irrigationsflüssigkeit zugeführt. Somit ist das chirurgische Handgerät durch die Konsole während des Eingriffs mit Ultraschallleistung, mit Irrigationsflüssigkeit sowie mit einem Unterdruck zu versorgen.

Des Weiteren ist denkbar, dass das augenchirurgische Gerätesystem zur Durchführung einer Vitrektomie dient. Hierbei wird ein als mechanischer Cutter ausgebildetes Handgerät in das Auge eingeführt. Zum Betrieb des Handgeräts wird dieses mit Druckluftimpulsen versorgt. Des Weiteren wird das geschnittene Glaskörpergewebe aus dem Auge abgesaugt. Somit ist das chirurgische Handgerät mit Druckluftimpulsen sowie mit Unterdruck zu versorgen.

Weitere mögliche Eingriffe, welche mit einem solchen Gerätesystem durchführbar sind, können beispielsweise eine Diathermie, beispielsweise eine Blepharoplastik, ein Eingriff mit Laserlicht, eine Ölinjektion etc. sein.

Entsprechende augenchirurgische Gerätesystem bieten somit eine Vielzahl von Verwendungsmöglichkeiten, die mit einer entsprechend hohen Anzahl von Einstellungsmöglichkeiten bzw. Parametern einhergehen, welche im Betrieb des Gerätesystems eingestellt und überwacht werden müssen. Dabei ist eine fehlerhafte Einstellung unbedingt zu vermeiden, da es sich bei dem Auge um ein äußerst empfindliches Organ handelt, so dass leicht schwere Verletzungen bis hin zu einer Erblindung bei einer fehlerhaften Handhabung auftreten können.

Der vorliegenden Erfindung liegt daher die Aufgabe zu Grunde, ein Verfahren zur Steuerung eines augenchirurgischen Gerätesystems anzugeben, bei dem auf einfache Weise eine sichere und fehlerfreie Steuerung ermöglicht wird. Des Weiteren soll ein augenchirurgisches Gerätesystem zur Durchführung eines solchen Verfahrens angegeben werden.

Erfindungsgemäß wird die voranstehende Aufgabe in verfahrensmäßiger Hinsicht durch die Merkmale des Anspruches 1 gelöst. Danach weist das in Rede stehende Verfahren zur Steuerung eines augenchirurgischen Gerätesystems, das eine Konsole umfasst, die mindestens eine Recheneinrichtung und mindestens eine graphische Anzeigeeinrichtung aufweist, wobei mindestens ein chirurgisches Handgerät durch das Gerätesystem betreibbar und versorgbar ist, insbesondere mit Überdruck und/oder Unterdruck und/oder Ultraschallleistung und/oder Flüssigkeit und/oder Licht und/oder elektromagnetischer Energie und/oder elektrischer Energie, die folgenden Verfahrensschritte auf:
- Anzeigen mindestens eines Benutzerprofils innerhalb eines ersten Darstellungsmoduls auf der Anzeigeeinrichtung, wobei dem Benutzerprofil mindestens ein Operationsprofil zugeordnet ist,
- Auswahl des gewünschten Benutzerprofils,
- Anzeige mindestens eines dem ausgewählten Benutzerprofil zugeordneten Operationsprofils innerhalb eines zweiten Darstellungsmoduls auf der Anzeigeeinrichtung, wobei dem Operationsprofil mindestens ein vordefiniertes Operationselement zugeordnet ist,
- Auswahl eines Operationsprofils,
- Anzeige mindestens eines dem Operationsprofil zugeordneten Operationselements innerhalb eines dritten Darstellungsmoduls auf der Anzeigeeinrichtung, wobei dem Operationselement mindestens ein Grundparameter des Gerätesystems zugeordnet ist,
- Auswahl eines Operationselementes, wobei der mindestens eine Grundparameter eingestellt bzw. berücksichtigt wird.

Es wird darauf hingewiesen, dass der Begriff "Steuerung" im weitesten Sinne zu verstehen ist, insbesondere den Betrieb des Gerätesystems umfasst.

Der Begriff "Ultraschallleistung" ist dahingehend zu verstehen, dass es ermöglicht wird, das Handgerät derart zu betreiben, dass das distale Ende zu einer Ultraschallschwingung angeregt wird. Beispielsweise kann ein Strom und/oder eine Spannung zur Verfügung gestellt werden, um in dem Handgerät angeordnete Piezoelemente in Schwingung zu versetzen.

Der Begriff "Flüssigkeit" umfasst insbesondere Öl und Irrigationsflüssigkeit, beispielsweise eine Kochsalzlösung. Weiterhin ist unter dem Begriff "Licht" sichtbares Licht zur Beleuchtung sowie Laserlicht zu verstehen. Der Begriff "elektrische Energie" beschreibt beispielsweise elektrischen Strom und/oder elektrische Spannung, mit der insbesondere ein Handgerät zur Beleuchtung oder ein Handgerät zur Diathermie versorgbar ist. Weiterhin kann das Gerätesystem "elektromagnetische Energie" beispielsweise für den Betrieb eines elektromagnetischen Vitrektors zur Verfügung stellen.

Des Weiteren kann das Handgerät Teil des Gerätesystems sein, wobei dies nicht zwangsweise der Fall ist.

In erfindungsgemäßer Weise ist zunächst erkannt worden, dass die Steuerung bzw. der Betrieb des Gerätesystems erheblich vereinfacht und sicherer gestaltet werden kann, indem für die Benutzer der Vorrichtung unterschiedliche Benutzerprofile existieren, wobei den einzelnen Benutzerprofilen jeweils Operationsprofile zugeordnet sind. Beispielsweise kann bei mehreren Operateuren jeder Operateur die Operationen angeben, die er mit dem Gerätesystem durchführt. Beispielsweise führt Benutzer A lediglich Phakoemulsifikationen durch, wohingegen Benutzer B Phakoemulsifikationen und Vitrektomien durchführt. Nach dem Einschalten des Gerätesystems, was beispielsweise über einen vorzugsweise kapazitiven oder induktiven Schalter erfolgen kann, wird vor der Durchführung einer Operation aus den innerhalb eines ersten Darstellungsmoduls angezeigten Benutzerprofilen derjenige Benutzer ausgewählt werden, der zu diesem Zeitpunkt das Gerätesystem verwendet. Bei der Anzeigeeinrichtung kann es sich beispielsweise um einen Monitor bzw. Display handeln. Alternativ oder zusätzlich kann eine mobile Anzeigeeinrichtung, insbesondere in der Art einer Fernbedienung, vorgesehen sein, beispielsweise ein Smartphone, ein Tablet-Computer oder ein PDA (Personal Digital Assistent).

Sodann werden die diesem Benutzerprofil zugeordneten Operationsprofile in einem zweiten Darstellungsmodul angezeigt. Wie voranstehend bereits ausgeführt kann es sich bei den Operationsprofilen beispielsweise um eine Phakoemulsifikation, um eine Vitrektomie oder einen beliebigen anderen ophthalmologischen Eingriff handeln. Des Weiteren ist denkbar, dass in Abhängigkeit von einer beliebigen Indikation des Patienten mehrere unterschiedliche Operationsprofile vorgesehen sind, beispielsweise eine Emulsifikation der natürlichen Linse verschiedener Ausprägungsgrade des diagnostizierten Katarakts. Je nachdem wie hart der zu entfernende Linsenkern ist, kann der Operateur das Operationsprofil wählen, bei dem die Operationselemente bzw. Operationsschritte und die Grundeinstellungen auf die Indikation, d.h. die Beschaffenheit der Linse abgestimmt sind.

In weiter erfindungsgemäßer Weise sind jedem Operationsprofil des jeweiligen Benutzers Operationselemente, insbesondere Operationsschritte, zugeordnet. Nach der Auswahl eines Operationsprofils, beispielsweise einer Phakoemulsifikation, werden in einem dritten Darstellungsmodul die für diesen Benutzer bei diesem Operationsprofil hinterlegten Operationselemente, das heißt Operationsschritte, angezeigt.

In weiter erfindungsgemäßer Weise sind für den ausgewählten Benutzer bei dem ausgewählten Operationsprofil und dem ausgewählten Operationselement Grundparameter zur Steuerung bzw. zum Betreiben der Vorrichtung vorgegeben. Hierbei kann es sich insbesondere um Maximalwerte der wesentlichen Betriebsparameter bzw. Grundparameter handeln.

Durch das erfindungsgemäße Verfahren ist es auf besonders einfache Weise und somit unter Vermeidung von Fehleinstellungen möglich, dass das augenchirurgische Gerätesystem mit den für eine bestimmte Operation und von einem bestimmten Operateur bevorzugten Grundparametern betrieben wird.

Es ist denkbar, dass im ersten Darstellungsmodul ein standard Benutzerprofil vorgesehen ist, dem mindestens ein Operationsprofil, insbesondere die Operationsprofile aller mit dem Gerätesystem durchführbaren Operationen, mit mindestens einem sicheren standard Grundparameter zugeordnet sind. Das standart Benutzerprofil kann vorzugsweise nicht löschbar sein. Sollte beispielsweise versehentlich ein Benutzerprofil gelöscht worden sein, kann der Operateur das standart Profil auswählen, um den Eingriff durchzuführen.

In besonders vorteilhafter Weise werden das erste, das zweite und das dritte Darstellungsmodul gleichzeitig auf der Anzeigeeinrichtung dargestellt. Dadurch ist es möglich, dass auf eine besonders einfache und sichere Weise eine Parametrierung des Gerätesystems durchführbar ist.

Des Weiteren ist denkbar, dass nach der Auswahl des Operationselements der mindestens eine Grundparameter in einem Grundparameter-Darstellungsmodul auf der Anzeigeeinrichtung angezeigt wird. Dadurch wird erreicht, dass die von dem Anwender zu erfassenden Informationen auf ein Minimum reduziert werden, nämlich lediglich die für die Durchführung des Operationselements bzw. Operationsschritts notwendigen Grundparameter dargestellt werden.

Um eine einfache Bedienung zu ermöglichen, könnte die Auswahl des Benutzerprofils, des Operationsprofils, des Operationselements und/oder eine Veränderung der Grundparametereinstellung über eine als Touchscreen ausgebildete Anzeigeeinrichtung erfolgen. Weiterhin ist es möglich, dass eine Multitouchsteuerung bzw. Gestensteuerung durch die Anzeigeeinrichtung ermöglicht wird und/oder dass die Darstellungsmodule, insbesondere mittels Multitouchsteuerung bzw. Gestensteuerung verschiebbar sind. Alternativ oder zusätzlich könnte die Auswahl über einen mit der Konsole gekoppelten Fußschalter durchgeführt werden. Der Fußschalter könnte in besonders vorteilhafter Weise batteriebetrieben oder akkubetrieben ausgebildet sein und mit der Konsole kabellos kommunizieren, bspw. über Bluetooth.

In weiter vorteilhafter Weise kann der Fußschalter ein Pedal und ggf. mehrere Tasten umfassen. Das Pedal könnte entlang der Längsachse, das heißt nach vorne und nach hinten, betätigbar sein. Des Weiteren ist denkbar, dass das Pedal drehbar ausgebildet ist und ggf. mehrere seitliche Tasten umfasst. Die Tasten könnten als Schaltwippen realisiert sein. In weiter vorteilhafter Weise könnten die mit den Pedal betätigbaren Funktionen über die Konsole veränderbar, das heißt die Belegung des Pedals anpassbar sein.

Gemäß einer vorteilhaften Weiterbildung des erfindungsgemäßen Verfahrens könnte nach einer Auswahl des Operationselements das Gerät initialisiert werden, beispielsweise eine mit dem Gerätesystem verbundene Schlauchverbindung mit Fluid gefüllt werden und/oder eine mit der Konsole verbundenes chirurgisches Handgerät mit einem Funktionstest unterzogen werden und/oder ein Erfassung der Ultraschallzeit zurückgesetzt werden und/oder eine Irrigationsquelle angeschlossen werden. Hierzu könnte auf der Anzeigeeinrichtung ein Initialisierungsmodul, vorzugsweise gleichzeitig mit der bzw. den Grundparameter-Einstellungsmodulen, dargestellt und von dem Anwender ausgewählt werden, so dass die notwendigen Initialisierungen dargestellt und ausgewählt werden können.

Bei den Grundparametern, die den Operationselementen des Benutzerprofils eines bestimmten Benutzers zugeordnet sind, kann es sich um eine Ultraschallleistung und/oder um eine Schnittrate eines Vitrektors und/oder um einen Unterdruck und/oder um eine Flussrate und/oder um eine Wärmeleistung und/oder um eine Lichtleistung handeln. Alternativ oder zusätzlich könnte es sich um einen Druck und/oder um eine Laserleistung und/oder um eine Laserpulsdauer und/oder um eine Dauer einer Laserpulspause und/oder um eine Intensität eines Zielstrahls handeln. Der Zielstrahl ermöglicht es dem Operateur zunächst mit sichtbarem Licht relativ geringer Intensität anzuzeigen, auf welchen Bereich des Gewebes das eigentlich anzuwendende Laserlicht fallen wird. Beispielsweise kann es sich bei dem Zielstrahl um sichtbares Laserlicht geringer Intensität handeln, wobei das Gerätesystem beispielsweise eine Umschaltung von dem als Zielstrahl dienenden Laserlicht auf den Laser ermöglichen kann, mit dem der eigentliche Eingriff durchgeführt wird.

Zur weiteren Verbesserung einer einfachen Steuerung und Handhabung des augenchirurgischen Gerätesystems könnte jeder für ein Operationselement voreingestellte Grundparameter während der Durchführung des Operationselements bzw. Operationsschritts in einem separaten Grundparameter-Darstellungsmodul angezeigt werden. Alternativ oder zusätzlich ist es denkbar, dass miteinander zusammenhängende Grundparameter, beispielsweise einer Laserleistung und eine Laserpulsdauer, in einem gemeinsamen Grundparameter-Darstellungsmodul angezeigt werden.

Zur Optimierung der Handhabbarkeit könnte während der Durchführung eines Operationselements ein maximaler Wert eines Grundparameters veränderbar sein bzw. verändert werden, beispielsweise über eine als Touchscreen ausgebildete Anzeigeeinrichtung oder über einen mit der Konsole gekoppelten Fußschalter. Beispielsweise könnte die maximale Ultraschallleistung während der Durchführung des Operationselements von dem Benutzer bzw. Operateur erhöht werden, wenn es sich - ggf. entgegen der ursprünglichen Annahme - um einen besonders harten Linsenkern handelt.

Gemäß einer weiteren Ausgestaltung ist denkbar, dass das Benutzerprofil und/oder das Operationsprofil und/oder das Operationselement neu angelegt, gelöscht und/oder verändert werden können.

Um eine besonders sichere Funktion des Gerätesystems zu gewährleisten, könnte - beispielsweise über einen oder mehrere Sensoren - die Funktion des Gerätesystems überwacht werden und bei einer Fehlfunktion ein entsprechender Fehlerhinweis auf der Anzeigeeinrichtung angezeigt werden. Im Konkreten könnten die möglichen Fehlfunktionen in mindestens zwei unterschiedliche Schweregrade eingeteilt sein und die Darstellung des Fehlerhinweises in Abhängigkeit des Schweregrads angepasst werden. Beispielsweise könnte bei einer Unterschreitung eines vorgegebenen Ladungszustands der Batterie des Fußschalters ein "leichter" Fehlerhinweis gegeben werden, dass die Fußschalterbatterie zu laden ist. Sofern der Ladezustand der Batterie bzw. des Akkus des Fußschalters einen Minimalwert unterschreitet, könnte ein "schwerer" Fehlerhinweis angezeigt werden, so dass der Anwender aufgefordert wird, die Batterie zu laden bzw. eine Verbindung zu einer externen Stromquelle herzustellen. Die Schweregrade könnten farblich gekennzeichnet sein, beispielweise grün bei einem reinen Hinweis, gelb bei einem leicht zu behebenden Fehler und rot oder blau bei einer schweren Fehlfunktion.

Zur weiteren Verbesserung der Handhabung könnte der Fehlerhinweis in dem die Fehlfunktion betreffenden Grundparameter-Darstellungsmodul angezeigt werden. Beispielsweise könnte bei einer zu hohen Spannung an einem Ultraschallhandgriff ein entsprechender Fehlerhinweis in dem Grundparameter-Darstellungsmodul der Ultraschallleistung erfolgen.

Gemäß einer besonders vorteilhaften Ausgestaltung des erfindungsgemäßen Verfahrens könnte während und/oder nach dem Betrieb des Gerätesystems ein Operationsbericht auf einem Speichermedium abgespeichert werden. Der Operationsbericht könnte beispielsweise das Benutzerprofil, die Operationsdauer, das Operationsprofil, die Ultraschalldauer und/oder etwaige Fehlerhinweise umfassen.

Die zugrundeliegende Aufgabe wird des Weiteren durch ein augenchirurgisches Gerätesystem gelöst, das gemäß Anspruch 15 zur Durchführung des erfindungsgemäßen Verfahrens dient.

Es wird darauf hingewiesen, dass das erfindungsgemäße Verfahren auch vorrichtungsmäßige Merkmale aufweist, die von dem erfindungsgemäßen Gerätesystem realisiert sein können und ausdrücklich einen Teil der hier beschriebenen Offenbarung darstellen. Das erfindungsgemäße augenchirurgische Gerätesystem kann somit die voranstehend sowie in der nachfolgenden Figurenbeschreibung in Bezug auf das Verfahren beschriebenen Merkmale aufweisen.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Darstellung ein Ausführungsbeispiel eines augenchirurgischen Gerätesystems zur Durchführung des erfindungsgemäßen Verfahrens,
- Fig. 2: in einer schematischen Darstellung die Anzeigeeinrichtung eines Gerätesystems zur Durchführung des Verfahrens, und
- Fig. 3: in einer schematischen Darstellung die Anzeigeeinrichtung eines Gerätesystems zur Durchführung des Verfahrens.

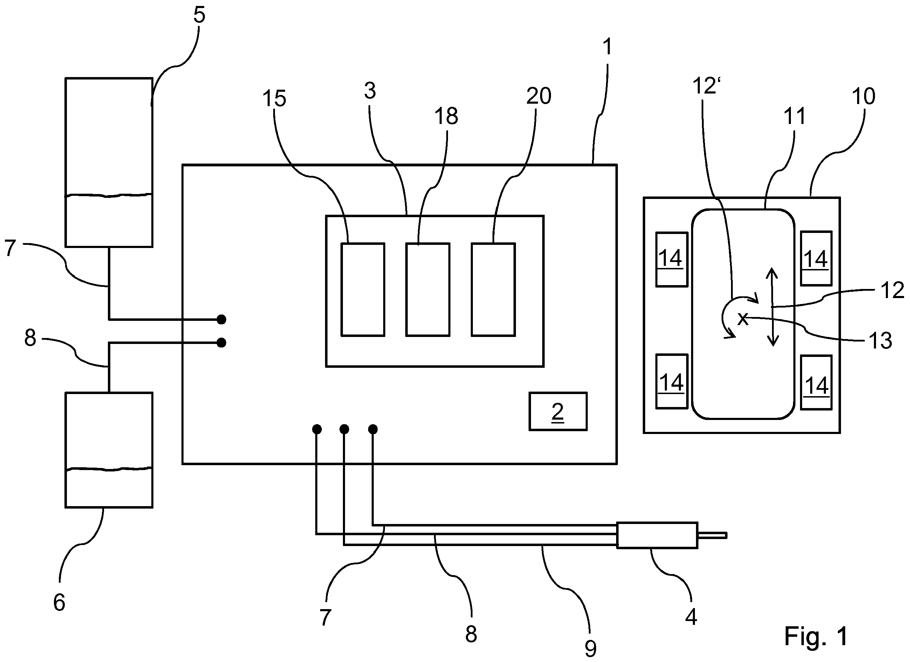

Fig. 1 zeigt ein Ausführungsbeispiel eines augenchirurgischen Gerätesystems zur Durchführung des erfindungsgemäßen Verfahrens. Das Gerätesystem umfasst eine Konsole 1 mit einer Recheneinrichtung 2. Bei der Recheneinrichtung 2 kann es sich beispielweise um einen Computer handeln, der zur Steuerung und/oder Regelung der verschiedenen Komponenten dient, die Teil des Gerätesystems sind bzw. mit diesem wirkverbunden sind. Des Weiteren umfasst das Gerätesystem eine graphische Anzeigeeinrichtung 3, beispielsweise einen Bildschirm bzw. Display.

Das Gerätesystem dient dazu, ein chirurgisches Handgerät 4 zu betreiben und zu versorgen, beispielsweise mit Überdruck, Unterdruck, Ultraschalleistung, Flüssigkeit, Licht, elektromagnetischer Energie und/oder elektromagnetischer Energie. In dem dargestellten Ausführungsbeispiel ist des Weiteren eine Flüssigkeitsquelle 5 gezeigt, über die das Handgerät 4 beispielsweise mit einer Kochsalzlösung versorgt werden kann. Die Flüssigkeitsquelle 5 kann durch einen Infusionsbehälter realisiert sein, der vorzugsweise an einer höhenverstellbaren Aufnahme festlegbar ist, um den Infusionsdruck bzw. Irrigationsdruck anzupassen. Zusätzlich kann das Gerätesystem eine nicht dargestellte Pumpeinrichtung aufweisen, so dass Flüssigkeit und ggf. Gewebeteile über das Handgerät 4 aus dem Auge in einen Auffangbehälter 6 abgesaugt werden können.

Das Handgerät 4 ist über eine Irrigationsschlauchverbindung 7 und einen Aspirationsschlauchverbindung 8 mit der Konsole 1 bzw. der Flüssigkeitsquelle 5 und dem Auffangbehälter 6 verbunden. Des Weiteren ist eine Leistungsverbindung 9 vorgesehen, um das Handgerät 4 bzw. die darin angeordnete Einrichtung - beispielsweise Piezo-Elemente - mit Ultraschallleistung zu versorgen.

Die Bedienung der Gerätesystems kann beispielsweise über die Anzeigeeinrichtung 3 erfolgen, wenn diese als Touchscreen ausgebildet ist. Des Weiteren kann ein Fußschalter 10 mit dem Gerätesystem gekoppelt sein bzw. einen Teil des Gerätesystems darstellen. Der Fußschalter 10 kann beispielsweise drahtlos, insbesondere über eine Bluetooth-Verbindung mit der Konsole 1 kommunizieren. In idealer Weise kann der Fußschalter 10 des Weiteren batteriebetrieben sein. Um einen Ausfall bei einem Versagen der Batterie zu vermeiden, könnte der Fußschalter 10 des Weiteren über ein Kabel mit einer externen Energiequelle und/oder mit der Konsole 1 verbunden werden.

Der Fußschalter 10 weist ein Pedal 11 auf, das entlang des Pfeils 12 kippbar und um die senkrecht auf der Bildebene stehende Achse 13 entlang des Pfeils 12' drehbar ist. Des Weiteren sind vier Tasten 14 vorgesehen.

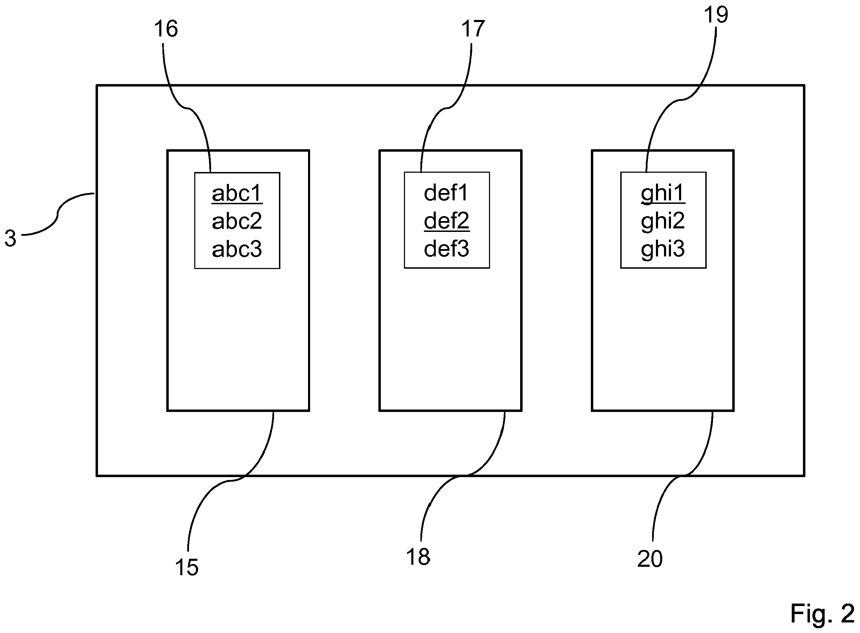

Fig. 2 zeigt in einer schematischen Darstellung die Anzeigeeinrichtung 3, an der die Durchführung des erfindungsgemäßen Verfahrens darstellbar ist.

Auf der Anzeigeeinrichtung 3 wird in einem ersten Darstellungsmodul 15 bzw. Fenster mindestens ein Benutzerprofil 16 dargestellt. Jedem Benutzerprofil 16 ist jeweils mindestens ein Operationsprofil 17 zugeordnet.

Nach der Auswahl des Benutzerprofils 16 wird in einem zweiten Darstellungsmodul 18 bzw. Fenster das mindestens eine zu diesem Benutzerprofil hinterlegte Operationsprofil 17 angezeigt. Hierbei könnte es sich um die Operationen handeln, die von dem ausgewählten Benutzer durchgeführt werden, beispielsweise eine Phakoemulsifikation und eine Diathermie. Dem mindestens einen Operationsprofil 17 ist mindestens ein vordefiniertes Operationselement 19 zugeordnet. Bei dem Operationselement 19 kann es insbesondere um einen Operationsschritt handeln.

Im hier dargestellten Ausführungsbeispiel ist in dem ersten Darstellungsmodul 15 der Benutzer "abc1" ausgewählt worden. Im zweiten Darstellungsmodul 18 ist das zu *"*abc1" hinterlegte Operationsprofil 17 "def2" im zweiten Darstellungsmodul 18 ausgewählt worden. Im dritten Darstellungsmodul 20 ist sodann das erste Operationsprofil 17 "ghi1" ausgewählt worden, welchem mindestens ein Grundparameter zugeordnet ist. Dieser Grundparameter wird bei der nachfolgenden Operation berücksichtigt. Bei dem Grundparameter kann es sich beispielsweise um eine maximale Ultraschallleistung handeln, mit der das Handgerät 4 betreibbar ist.

Durch das erfindungsgemäße Verfahren ist es somit möglich, dass das augenchirurgische Gerätesystem aus besonders einfache Weise mit den von dem Benutzer präferierten Einstellungen betreibbar ist, wobei durch die gleichzeitige Darstellung des ersten Darstellungsmoduls 15, des zweiten Darstellungmoduls 18 und des dritten Darstellungsmoduls 20 für die Anwender der Vorrichtung auf besonders einfache Weise nachvollziehbar ist, dass die Einstellungen des Gerätes korrekt sind.

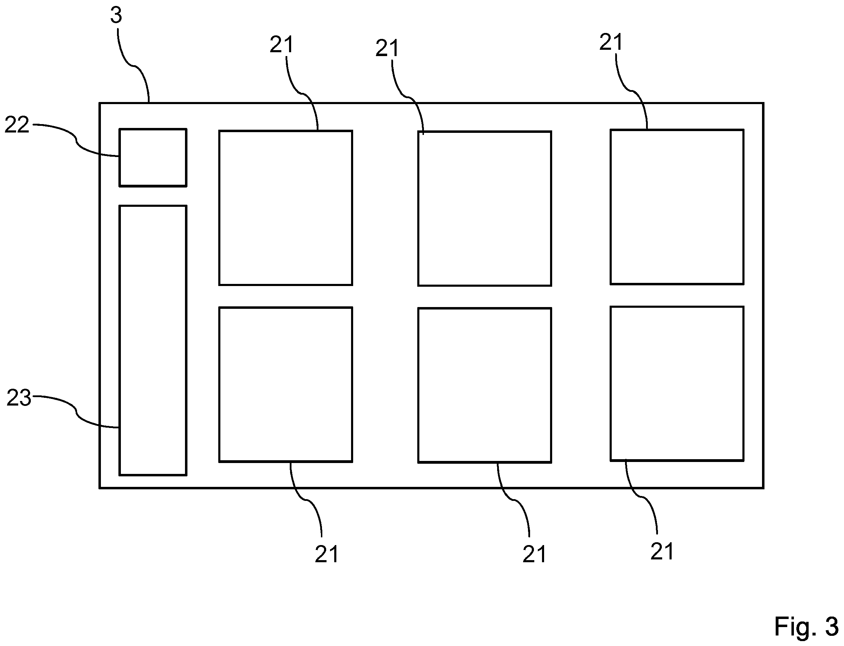

Fig. 3 zeigt in einer schematischen Darstellung die Anzeigeeinrichtung 3 nach der Auswahl des Operationselements 19.

Dabei werden die für die Durchführung des Operationselements 19 relevanten Grundparameter jeweils in einem Grundparameter-Darstellungsmodul 21 dargestellt. Somit können alle relevanten Grundparameter angezeigt und gegebenenfalls verändert werden, beispielsweise mittels einer als Touchscreen ausgebildeten Anzeigeeinrichtung 3 und/oder den Fußschalter 10.

Des Weiteren kann ein Initialisierungsmodul 22 dargestellt werden, das es dem Benutzer ermöglicht, nach der Auswahl des Operationselements 19 das Gerätesystem zu initialisieren. Beispielsweise kann eine der Schlauchverbindung 7, 8 mit einem Fluid befüllt werden und/oder das Handgerät 4 einem Funktionstest unterzogen werden und/oder eine Erfassung der Ultraschallzeit einer vorhergegangenen Operation zurückgesetzt werden und/oder die Flüssigkeitsquelle 5 bzw. Irrigationsquelle angeschlossen und gegebenenfalls an ihre Höhe angepasst werden.

Gemäß einer weiteren vorteilhaften Ausgestaltung des erfindungsgemäßen Verfahren ist es denkbar, dass bei einer Fehlfunktion ein entsprechender Fehlerhinweis auf der Anzeigeeinrichtung 3 angezeigt wird, wobei der Fehlerhinweis in dem die Fehlfunktion betreffenden Grundparameter-Darstellungsmodul 21 erscheinen kann. Somit ist für den Anwender unmittelbar nachvollziehbar, welcher Bestandteil des Gerätesystems eine Fehlfunktion aufweist und überprüft werden muss.

Zusätzlich sind in einem Operationselemente-Darstellungsmodul 23 die zu dem Operationsprofil 17 - beispielsweise einer Vitrektomie - zugeordneten Operationselemente 19 dargestellt, wobei das aktuell ausgeführte Operationselement 19 kenntlich gemacht sein kann.

Hinsichtlich weiterer vorteilhafter Ausgestaltungen des erfindungsgemäßen Verfahrens und der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele des erfindungsgemäßen Verfahrens und der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Konsole
- 2: Rechenrichtung
- 3: Anzeigeeinrichtung
- 4: Handgerät
- 5: Flüssigkeitsquelle
- 6: Auffangbehälter
- 7: Irrigationsschlauchverbindung
- 8: Aspirationsschlauchverbindung
- 9: Leistungsverbindung
- 10: Fußschalter
- 11: Pedal
- 12, 12': Pfeil
- 13: Achse
- 14: Taste
- 15: erstes Darstellungsmodul
- 16: Benutzerprofil
- 17: Operationsprofil
- 18: zweites Darstellungsmodul
- 19: Operationselement
- 20: drittes Darstellungsmodul
- 21: Grundparameter-Darstellungsmodul
- 22: Initialisierungsmodul
- 23: Operationselemente-Darstellungsmodul

## Patentansprüche

1. Verfahren zur Steuerung eines augenchirurgischen Gerätesystems, das eine Konsole (1) umfasst, die mindestens eine Recheneinrichtung (2) und mindestens eine graphische Anzeigeeinrichtung (3) aufweist, wobei mindestens ein chirurgisches Handgerät (4) durch das Gerätesystem betreibbar und versorgbar ist, insbesondere mit Überdruck und/oder Unterdruck und/oder Ultraschallleistung und/oder Flüssigkeit und/oder Licht und/oder elektromagnetischer Energie und/oder elektrischer Energie, mit folgenden Verfahrensschritten:
- Anzeigen mindestens eines Benutzerprofils (16) innerhalb eines ersten Darstellungsmoduls (15) auf der Anzeigeeinrichtung (3), wobei dem Benutzerprofil (16) mindestens ein Operationsprofil (17) zugeordnet ist,
- Auswahl des gewünschten Benutzerprofils (16),
- Anzeige mindestens eines dem ausgewählten Benutzerprofil (16) zugeordneten Operationsprofils (17) innerhalb eines zweiten Darstellungsmoduls (18) auf der Anzeigeeinrichtung (3), wobei dem Operationsprofil (17) mindestens ein vordefiniertes Operationselement (19) zugeordnet ist,
- Auswahl eines Operationsprofils (17),
- Anzeige mindestens eines dem Operationsprofil (17) zugeordneten Operationselements (19) innerhalb eines dritten Darstellungsmoduls (20) auf der Anzeigeeinrichtung (3), wobei dem Operationselement (19) mindestens ein Grundparameter des Gerätesystems zugeordnet ist,
- Auswahl eines Operationselementes (19), wobei der mindestens eine Grundparameter eingestellt bzw. berücksichtigt wird.

2. Verfahren nach Anspruch 1, **dadurch gekennzeichnet, dass** das erste, das zweite und das dritte Darstellungsmodul (15, 18, 20) gleichzeitig auf der Anzeigeeinrichtung (3) dargestellt werden.

3. Verfahren nach Anspruch 1 oder 2, **dadurch gekennzeichnet, dass** nach der Auswahl des Operationselementes (19) der mindestens eine Grundparameter in einem Grundparameter-Darstellungsmodul (21) auf der Anzeigeeinrichtung (3) angezeigt wird.

4. Verfahren nach einem der Ansprüche 1 bis 3, **dadurch gekennzeichnet, dass** die Auswahl über die als Touchscreen ausgebildete Anzeigeeinrichtung (3) und/oder über einen mit der Konsole (1) gekoppelten Fußschalter (10) erfolgt.

5. Verfahren nach einem der Ansprüche 1 bis 4, **dadurch gekennzeichnet, dass** ein mit der Konsole (1) gekoppelter Fußschalter (10) angeordnet ist, wobei der Fußschalter (10) ein Pedal (11) und ggf. mehrere Tasten (14) umfasst und wobei die Funktion des Pedals (11) und/oder ggf. einiger der Tasten (14) über die Konsole (1) verändert werden kann.

6. Verfahren nach einem der Ansprüche 1 bis 5, **dadurch gekennzeichnet, dass** nach der Auswahl des Operationselements (19) das Gerätesystem initialisiert wird, beispielsweise eine mit dem Gerätesystem verbundene Schlauchverbindung (7, 8) mit einem Fluid gefüllt wird und/oder ein mit dem Gerätesystem verbundenes chirurgisches Handgerät (4) einem Funktionstest unterzogen wird und/oder eine Erfassung der Ultraschallzeit zurückgesetzt wird und/oder eine Flüssigkeitsquelle (5) angeschlossen wird.

7. Verfahren nach einem der Ansprüche 1 bis 6, **dadurch gekennzeichnet, dass** es sich bei dem Grundparameter um eine Ultraschalleistung und/oder um eine Schnittrate und/oder um einen Unterdruck und/oder um eine Flussrate und/oder um eine Wärmeleistung und/oder um eine Lichtleistung und/oder um einen Druck und/oder um eine Laserleistung und/oder um eine Laserpulsdauer und/oder um eine Dauer einer Laserpulspause und/oder um eine Zielstrahlintensität handelt.

8. Verfahren nach einem der Ansprüche 1 bis 7, **dadurch gekennzeichnet, dass** jeder für ein Operationselement (19) voreingestellte Grundparameter während der Durchführung des Operationselements (19) in einem separaten Grundparameter-Darstellungsmodul (21) angezeigt wird.

9. Verfahren nach einem der Ansprüche 1 bis 8, **dadurch gekennzeichnet, dass** während der Durchführung eines Operationselementes (19) ein Maximalwert eines Grundparameters verändert werden kann, beispielsweise über die als Touchscreen ausgebildete Anzeigeeinrichtung (3) und/oder über einen mit der Konsole (1) gekoppelten Fußschalter (10).

10. Verfahren nach einem der Ansprüche 1 bis 9, **dadurch gekennzeichnet, dass** das Benutzerprofil (16) bzw. die Benutzerprofile (16) und/oder das Operationsprofil (17) bzw. die Operationsprofile (17) und/oder das Operationselement (19) bzw. die Operationselemente (19) neu angelegt und/oder gelöscht und/oder verändert werden können.

11. Verfahren nach einem der Ansprüche 1 bis 10, **dadurch gekennzeichnet, dass** die Funktion des Gerätesystems, insbesondere über einen oder mehrere Sensoren, überwacht wird und dass bei einer Fehlfunktion ein entsprechender Fehlerhinweis auf der Anzeigeeinrichtung (3) angezeigt wird.

12. Verfahren nach Anspruch 11, **dadurch gekennzeichnet, dass** die möglichen Fehlfunktionen in mindestens zwei unterschiedliche Schweregrade eingeteilt werden und dass der Fehlerhinweis in Abhängigkeit des Schweregrads angepasst wird.

13. Verfahren nach einem der Ansprüche 11 oder 12, **dadurch gekennzeichnet, dass** der Fehlerhinweis in einem die Fehlfunktion betreffenden Grundparameter-Darstellungsmodul (21) angezeigt wird.

14. Verfahren nach einem der Ansprüche 1 bis 13, **dadurch gekennzeichnet, dass** während und/oder nach dem Betrieb des Gerätesystems ein Operationsbericht auf einem Speichermedium abgespeichert wird, der beispielsweise das Benutzerprofil, die Operationsdauer, das Operationsprofil, die Ultraschalldauer und/oder Fehlerhinweise umfasst.

15. Augenchirurgisches Gerätesystem zur Durchführung des Verfahrens nach einem der Ansprüche 1 bis 14.
